# EUROPEAN PATENT SPECIFICATION

(11) **EP 3 515 438 B1**
(45) Date of publication and mention of the grant of the patent: **02.03.2022**
(21) Application number: 17853697.5
(22) Date of filing: 15.09.2017
(51) Int. Cl.: A61K 9/20, A61K 9/00, A61K 45/06, A61K 31/4375, A61M 5/00, A61K 31/436, A61P 31/18

(54) **DRUG DELIVERY SYSTEM FOR THE DELIVERY OF INTEGRASE INHIBITORS**
WIRKSTOFFFREISETZUNGSSYSTEM ZUR FREISETZUNG VON INTEGRASEINHIBITOREN
SYSTÈME D'ADMINISTRATION DE MÉDICAMENTS POUR L'ADMINISTRATION D'INHIBITEURS DE L'INTÉGRASE

(30) Priority: 21.09.2016 US 201662397618 P
(43) Date of publication of application: 31.07.2019
(73) Proprietor: Merck Sharp & Dohme Corp., Rahway, NJ 07065-0907 (US)
(72) Inventor: BARRETT, Stephanie, E., West Point Pennsylvania 19486 (US); GINDY, Marian, West Point Pennsylvania 19486 (US); HENRY, Steven, J., Ephrata Pennsylvania 17522 (US); FORSTER, Seth, P., West Point Pennsylvania 19486 (US); TELLER, Ryan, S., West Point Pennsylvania 19486 (US); GROBLER, Jay, A., West Point Pennsylvania 19486 (US)
(74) Representative: Jaap, David Robert
(86) International application number: PCT/US2017/051686
(87) International publication number: WO 2018/057408

(56) References cited:
- WO-A1-94/28871
- WO-A1-2018/140368
- US-A1- 2008 161 271
- US-A1- 2009 318 421
- US-A1- 2016 228 419
- Malay Das ET AL: "Progress in Brain Delivery of Anti-HIV Drugs", Journal of Applied Pharmaceutical Science, 1 January 2015 (2015-01-01), pages 154-164, XP055689539, DOI: 10.7324/JAPS.2015.50724 Retrieved from the Internet: URL:https://www.japsonline.com/admin/php/u ploads/1569_pdf.pdf

## Description

### BACKGROUND OF THE INVENTION

The development of highly active antiretroviral therapy (HAART) in the mid 1990's transformed the clinical care of human immunodeficiency virus (HIV) type infection. HAART regimens have proven to be highly effective treatments, significantly decreasing HIV viral load in HIV-infected patients, thereby slowing the evolution of the illness and reducing HIV-related morbidity and mortality. Yet, the treatment success of HAART is directly related to adherence to the regimen by the patient. Unless appropriate levels of the antiretroviral drug combinations are maintained in the blood, viral mutations will develop, leading to therapy resistance and cross-resistances to molecules of the same therapeutic class, thus placing the long-term efficacy of treatments at risk. Various clinical studies have shown a decline in treatment effectiveness with relatively small lapses in adherence. A study by Musiime found that 81% of patients with more than 95% adherence demonstrated viral suppression, while only 50% of patients who were 80-90% adherent were successful. See, Musiime, S., et al., Adherence to Highly Active Antiretroviral Treatment in HIV-Infected Rwandan Women. PLOS one 2011, 6, (11), 1-6. Remarkably, only 6% of patients that were less than 70% adherent showed improvements in viral markers. Thus, low adherence is a leading cause of therapeutic failure in treatment of HIV-1 infection.

Nonetheless, adherence rates to the HAART regimens continue to be far from optimal. Various characteristics of HAART make adherence particularly difficult. Therapeutic regimens are complex, requiring multiple drugs to be taken daily, often at different times of the day, and many with strict requirements on food intake. Many HAART medications also have unpleasant side effects, including nausea, diarrhea, headache, and peripheral neuropathy. Social and psychological factors can also negatively impact adherence. Patients report that forgetfulness, lifestyle factors, including fear of being identified as HIV-positive, and therapy fatigue over life-long duration of treatment all contribute to adherence lapses.

New HIV treatment interventions aim to improve adherence by reducing the complexity of treatments, the frequency of the dosages, and/or the side effects of the medications. Long-acting injectable (LAI) drug formulations that permit less frequent dosing, on the order of a month or longer, are an increasingly attractive option to address adherence challenges. However, the majority of approved and investigational antiretroviral agents are not well suited for reformulation as long-acting injectable products. In large part, this is due to suboptimal physicochemical properties limiting their formulation as conventional drug suspensions, as well as insufficient antiviral potency resulting in high monthly dosing requirements. Even for cabotegravir or rilpivirine, two drugs being studied as long-acting injectible formulations, large injection volumes and multiple injections are required to achieve pharmacokinetic profiles supportive of monthly dosing. See, e.g., Spreen, W. R., et al., Long-acting injectable antiretrovirals for HIV treatment and prevention. Current Opinion in Hiv and Aids 2013, 8, (6), 565-571; Rajoli, R. K. R., et al., Physiologically Based Pharmacokinetic Modelling to Inform Development of Intramuscular Long-Acting Nanoformulations for HIV. Clinical Pharmacokinetics 2015, 54, (6), 639-650; Baert, L., et al., Development of a long-acting injectable formulation with nanoparticles of rilpivirine (TMC278) for HIV treatment. European Journal of Pharmaceutics and Biopharmaceutics 2009, 72, (3), 502-508; Van 't Klooster, G., et al., Pharmacokinetics and Disposition of Rilpivirine (TMC278) Nanosuspension as a Long-Acting Injectable Antiretroviral Formulation. Antimicrobial Agents and Chemotherapy 2010, 54, (5), 2042-2050.

Ramirez Garcia, P. et al., Factors affecting adherence to antiretroviral therapy in people living with HIV/AIDS. The Journal of the Association of Nurses in AIDS Care: JANAC 2003, 14, (4), 37-45, states that therapy adherence declines with the number of medications, the frequency of the dosages, and the increasing complexity and duration of the treatments. The complexity of the regimens, the large number of capsules that must be taken, the variety of dosages, the various interactions among the medications as well as between food and the medications may easily lead to confusion and thus to poor adherence. Whetten, K., et al. Trauma, mental health, distrust, and stigma among HIV-Positive persons: Implications for effective care. Psychosomatic Medicine 2008, 70, states that traumatic events, mental illness, distrust, and stigma associated with HIV have been linked with poorer adherence to medication regimens. Karmon, S.L., et al., Next-Generation Integrase Inhibitors: Where to After Raltegravir? Drugs 2013, 73, (3), 213-228, states that raltegravir, the first approved integrase strand transfer inhibitor, is a potent and well tolerated antiviral agent with limitations of twice-daily dosing.

Thus, novel formulation approaches capable of delivering extended-duration pharmacokinetic characteristics for integrase inhibitors of diverse physicochemical properties at practical injection volumes and with a limited number of injections are highly desirable.

### SUMMARY OF THE INVENTION

This invention relates to a pellet implant drug delivery system for long-acting delivery of an integrase inhibitor. The system is useful for the treatment or prevention of human immunodeficiency virus (HIV) infection.

### DETAILED DESCRIPTION OF THE INVENTION

This invention relates to an implant drug delivery system for long-acting delivery of the integrase inhibitor (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide.

The system comprises between 1 and 20 compressed pellets of this integrase inhibitor, wherein the diameter of said pellet is about 1 mm to about 5 mm.

The system is useful for the treatment or prevention of human immunodeficiency virus (HIV) infection. The invention further relates to this implant drug delivery system for use in treating or preventing HIV infection in a patient. (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide has the following chemical structure: 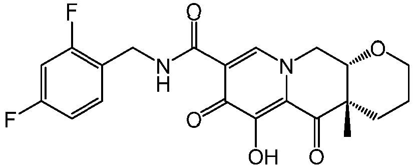 Preparation of and the ability of (4aS, 11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide to inhibit integrase are described in PCT Publication WO2015048363 (Example 10).

An additional (reference) integrase inhibitor is (3aS,5R)-N-(2,4-difluorobenzyl)-2-ethyl-8-hydroxy-5-methoxy-1,7-dioxo-2,3,3a,4,5,7-hexahydro-1H-pyrazino[2,1,6-cd]indolizine-6-carboxamide. (3aS,5R)-N-(2,4-difluorobenzyl)-2-ethyl-8-hydroxy-5-methoxy-1,7-dioxo-2,3,3a,4,5,7-hexahydro-1H-pyrazino[2,1,6-cd]indolizine-6-carboxamide has the following chemical structure:

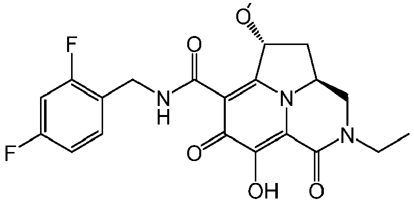

Preparation of and the ability of (3aS,5R)-N-(2,4-difluorobenzyl)-2-ethyl-8-hydroxy-5-methoxy-1,7-dioxo-2,3,3a,4,5,7-hexahydro-1H-pyrazino[2,1,6-cd]indolizine-6-carboxamide to inhibit integrase are described in PCT Publication WO2014183532 (Example 12).

In an embodiment of the invention, the novel implant delivery systems of the invention comprise between 1 and 20 compressed pellets comprising an integrase inhibitor, with or without a polymer coating to act as a rate controlling membrane. The compressed pellets can be sterilized in accordance to accepted sterilization practices for use *in vivo.* The sterile compressed pellets provide advantages beyond previously described implants, including high drug load capacity, greater dynamic range of drug input rate, flexibility on design (erodible and non-erodible), and removability. The pellet diameters are between about 1mm to about 5 mm, preferably about 1 mm to about 3 mm, and more preferable about 2 mm.

In an embodiment of the invention, pellet length is about 5 mm to about 50 mm, preferably about 6 mm to about 20 mm, more preferably about 7 mm to about 10 mm, e.g., about 7 mm, e.g., 7 mm, about 8 mm, e.g., 8 mm, about 9 mm, e.g., 9 mm, and about 10 mm, e.g., 10 mm.

An embodiment of the invention is an implant drug delivery system comprising subcutaneously implanting between 1 and 20 compressed pellets of (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide, wherein the diameter of said pellet is about 1 mm to about 5 mm for use in preventing HIV infection in a patient.

Depending on the size of the pellet, and the dose of drug required to deliver a therapeutically effective amount of active ingredient, a therapeutically effective dose of the active ingredient can be delivered by implanting a single pellet or multiple pellets, e.g. 2-20 pellets. More specifically, the pellet implant drug delivery system comprises 1, 2, 3, 4, 5, 6, 7, 8, 9 or 10 or more pellets.

Pellets can be prepared using a compression technique or a molding technique. Pellets are formed by compressing or molding active ingredient powder such as micronized powder, optionally using solubility enhancers to enhance solubility, lubricants to enhance processing of the formed pellets, emulsifiers, humectants and nonionic surfactants.

Solubilizing agents include but are not limited to polyethylene glycol, Poloxamer 407, propylene glycol, hydroxypropyl-β-cyclodextrin, sulfobutylether-β-cyclodextrin, α-cyclodextrin, phospholipids, castor oil, hydrogenated castor oil, solutol, sorbitan monooleate, sucrose, dextrose anhydrous, dextrose monohydrate, and mannitol and the like.

Lubricants include but are not limited to magnesium stearate, calcium stearate, sodium stearate, talc, sterotex, waxes, stearowet, glyceryl behapate, liquid paraffin and the like.

Emulsifiers include but are not limited to glyceryl monostearate, stearic acid, stearyl alcohol, cetyl alcohol, and the like.

Humectants include but are not limited to glycerol, ethylene glycol, polyethylene glycol (PEG), diethylene glycol, triethylene glycol, propylene glycol, dipropylene glycol, glycerin, sorbitol, mannitol, glucose, and the like.

Nonionic surfactants include but are not limited to POE (20) sorbitan monooleate, also known as polyethylene glycol sorbitan monooleate, polyoxyethylenesorbitan monooleate, Polysorbate 80, D-α-Tocopheryl polyethylene glycol 1000 succinate and Tween ^{®} 80, and the like.

In the compression technique, emulsifiers (e.g., glyceryl monostearate), solubility enhancers (e.g., polyethylene glycol, including PEG 6000) and humectants (e.g., glycerol) or nonionic surfactants (e.g., Tween 80 or Polysorbate 80) may optionally be used and heated (e.g., about 70 °C) on a water bath under stirring. The drug is dispersed uniformly just before the mass solidifies. The solidified blend is stored in freezer, and the hard mass is ground to fine powder and passed through a sieve (e.g., aperture 0.42 mm). The solidified blend can also be micronized using standard technology. Granules are compressed under pressure, for example at 4-10 kN, and at room temperature, on a rotary compression machine, such as a uniaxial mechanical tablet tooling, to form tablet shaped pellets of desired diameter. Different formulations can be prepared using various glycerol or Tween 80 concentrations.

In the molding technique, the molten mass and the various formulations are prepared as above, except that the molten mass is drawn up into a syringe and injected into a cylindrical stainless steel mold. The mold is allowed to cool and each cylindrical pellet is cut into pieces equivalent to those prepared by the compression technique, to make the same size pellets by both techniques.

Additionally, the implant drug delivery systems of the instant invention may be produced using an extrusion process, wherein ground biocompatible polymer is blended with the antiviral agent, melted and extruded into rod-shaped structures. Rods are cut into individual implantable devices of the desired length, packaged and sterilized prior to use. Implant drug delivery systems with a polymer coating to act as a rate controlling membrane may be formed, for example, by coextrusion. Specifically, the pellet is implanted subcutaneously and (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is continually released *in vivo* at a rate resulting in a plasma concentration between 0.1 ng/mL and 500,000.0 ng/mL. The pellet implant delivery system is desired and useful for prophylaxis and/or treatment of HIV infection from both compliance and convenience standpoints.

As used herein, the term "continually released" refers to the drug being released into plasma at continuous rates for extended periods of time. The implant drug delivery system of the instant invention generally exhibits linear release kinetics for the drug *in vivo,* sometimes after an initial burst.

Optionally, the pellet implant delivery system of the instant invention can further comprise a radioopaque component. The radioopaque component will cause the implant to be X-ray visible. The radioopaque component can be any such element known in the art, such as barium sulphate, titanium dioxide, bismuth oxide, tantalum, tungsten or platinum. In a specific embodiment, the radioopaque component is barium sulphate.

In an embodiment of the invention, the integrase inhibitor is administered as a monotherapy. In another embodiment of the invention, an integrase inhibitor and another antiviral agent are administered in combination.

An "anti-HIV agent" is any agent which is directly or indirectly effective in the inhibition of HIV reverse transcriptase or another enzyme required for HIV replication or infection, the or prophylaxis of HIV infection, and/or the treatment, prophylaxis or delay in the onset or progression of AIDS. It is understood that an anti-HIV agent is effective in treating, preventing, or delaying the onset or progression of HIV infection or AIDS and/or diseases or conditions arising therefrom or associated therewith. Suitable other anti-viral agents for use in implant drug delivery systems described herein include, for example, those listed in Table A as follows:

**Table A: Antiviral Agents for Preventing HIV infection or AIDS**

| Name | Type |
|---|---|
| abacavir, ABC, Ziagen^{®} | nRTI |
| abacavir +lamivudine, Epzicom^{®} | nRTI |
| abacavir + lamivudine + zidovudine, Trizivir^{®} | nRTI |
| amprenavir, Agenerase^{®} | PI |
| atazanavir, Reyataz^{®} | PI |
| AZT, zidovudine, azidothymidine, Retrovir^{®} | nRTI |
| Capravirine | nnRTI |
| darunavir, Prezista^{®} | PI |
| ddC, zalcitabine, dideoxycytidine, Hivid^{®} | nRTI |
| ddI, didanosine, dideoxyinosine, Videx^{®} | nRTI |
| ddI (enteric coated), Videx EC^{®} | nRTI |
| delavirdine, DLV, Rescriptor^{®} | nnRTI |
| efavirenz, EFV, Sustiva^{®}, Stocrin^{®} | nnRTI |
| efavirenz + emtricitabine + tenofovir DF, Atripla^{®} | nnRTI + nRTI |
| EFdA (4'-ethynyl-2-fluoro-2'-deoxyadenosine) | nRTI |
| emtricitabine, FTC, Emtriva^{®} | nRTI |
| emtricitabine + tenofovir DF, Truvada^{®} | nRTI |
| emvirine, Coactinon^{®} | nnRTI |
| enfuvirtide, Fuzeon^{®} | FI |
| enteric coated didanosine, Videx EC^{®} | nRTI |
| etravirine, TMC-125 | nnRTI |
| fosamprenavir calcium, Lexiva^{®} | PI |
| indinavir, Crixivan^{®} | PI |
| lamivudine, 3TC, Epivir^{®} | nRTI |
| lamivudine + zidovudine, Combivir^{®} | nRTI |
| Lopinavir | PI |
| lopinavir + ritonavir, Kaletra^{®} | PI |
| maraviroc, Selzentry^{®} | EI |
| nelfinavir, Viracept^{®} | PI |
| nevirapine, NVP, Viramune^{®} | nnRTI |
| PPL-100 (also known as PL-462) (Ambrilia) | PI |
| raltegravir, Isentress^{®} | InI |
| (S)-2-(3-chloro-4-fluorobenzyl)-8-ethyl-10-hydroxy- N,6-dimethyl-1,9-dioxo-1,2,6,7,8,9-hexahydropyrazino[1',2':1,5]pyrrolo[2,3-d]pyridazine-4-carboxamide (MK-2048) | InI |
| ritonavir, Norvir^{®} | PI |
| saquinavir, Invirase^{®}, Fortovase^{®} | PI |
| stavudine, d4T,didehydrodeoxythymidine, Zerit^{®} | nRTI |
| tenofovir DF (DF = disoproxil fumarate), TDF, Viread^{®} | nRTI |
| Tenofovir, hexadecyloxypropyl (CMX-157) | nRTI |
| tipranavir, Aptivus^{®} | PI |
| Vicriviroc | EI |

| | |
|---|---|
| EI = entry inhibitor; FI = fusion inhibitor; InI = integrase inhibitor; PI = protease inhibitor; nRTI = nucleoside reverse transcriptase inhibitor; nnRTI = non-nucleoside reverse transcriptase inhibitor. | |

Other suitable integrase inhibitors include Elvitegravir (U.S. Patent 7,176,220), BI 224436 ((2*S*)-[4-(3,4-Dihydro-2*H*-chromen-6-yl)-3-quinolinyl][(2-methyl-2-propanyl)oxy]acetic acid), Globoidnan A (Ovenden, et al., Phytochemistry 65 (2004) 3255-3259), Bictegravir (WO2014100323), Dolutegravir, Cabotegravir, (3aS,5R)-N-(2,4-difluorobenzyl)-2-ethyl-8-hydroxy-5-methoxy-1,7-dioxo-2,3,3a,4,5,7-hexahydro-1H-pyrazino[2,1,6-cd]indolizine-6-carboxamide, and (4aS,1 1aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide.

| | |
|---|---|
| Dolutegravir | 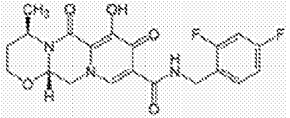 |
| | WO2006116764 |
| | WO2010068253 (compound AA) |
| Cabotegravir | 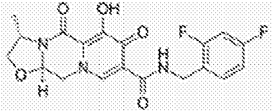 |
| | WO2006116764 (Example Z-1) |
| (3 aS, 5R)-N-(2,4-difluorobenzyl)-2-ethyl-8-hydroxy-5-methoxy-1,7-dioxo-2,3,3a,4,5,7-hexahydro-1H-pyrazino[2,1,6-cd]indolizine-6-carboxamide | 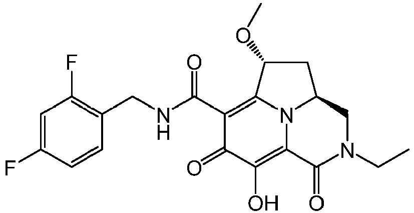 |
| | WO2014183532 A1 (Example 12) |
| (4aS,11aR)-N-(2,4-difluorobenzyl)-6-hydroxy-4a-methyl-5,7-dioxo-2,3,4,4a,5,7,11, 1 1a-octahydropyrano[3,2-b]quinolizine-8-carboxamide | 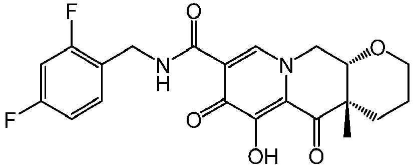 |
| | WO2015048363 (Example 10) |

Some of the drugs listed in the table can be used in a salt form, e.g., abacavir sulfate, delavirdine mesylate, indinavir sulfate, atazanavir sulfate, nelfinavir mesylate, saquinavir mesylate.

In certain embodiments the integrase inhibitors in the implant drug delivery system described herein are employed in their conventional dosage ranges and regimens as reported in the art, including, for example, the dosages described in editions of the Physicians' Desk Reference, such as the 63rd edition (2009) and earlier editions. In other embodiments, the integrase inhibitors in the implant drug delivery system described herein are employed in lower than their conventional dosage ranges. In other embodiments, the integrase inhibitors in the implant drug delivery system described herein are employed in higher than their conventional dosage ranges.

In an embodiment of the implant drug delivery system described herein, the integrase inhibitor is present in the pellet at about 0.10% - 100% by weight of drug loading. In other embodiments, the integrase inhibitor is present in the pellet at about 0.10%-99% by weight, at about 60%-99% by weight, at about 80%-99%, at about 90%-99% by weight, or at about 99% by weight of drug loading. In a class of the embodiment of the implant drug delivery system described herein, (4aS,11aR)-N-(2,4-difluorobenzyl)-6-hydroxy-4a-methyl-5,7-dioxo-2,3,4,4a,5,7,11,1 1a-octahydropyrano[3,2-b]quinolizine-8-carboxamide is present in the pellet at about 0.10%-100% by weight of drug loading. In a subclass of the embodiment of the implant drug delivery system described herein, (4aS,1 1aR)-N-(2,4-difluorobenzyl)-6-hydroxy-4a-methyl-5,7-dioxo-2,3,4,4a,5,7,11,1 1a-octahydropyrano[3,2-b]quinolizine-8-carboxamide is present in the pellet at about 0.10%-99% by weight of drug loading. In a further subclass of the embodiment of the implant drug delivery system described herein, (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is present in the pellet at about 60%-99% by weight of drug loading. In a further subclass of the embodiment of the implant drug delivery system described herein, (4aS,1 1aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is present in the pellet at about 80%-99% by weight of drug loading. In a further subclass of the embodiment of the implant drug delivery system described herein, (4aS,11aR)-N-(2,4-difluorobenzyl)-6-hydroxy-4a-methyl-5,7-dioxo-2,3,4,4a,5,7,11,1 1a-octahydropyrano[3,2-b]quinolizine-8-carboxamide is present in the pellet at about 90%-99% by weight of drug loading. In an example of the embodiment of the implant drug delivery system described herein, (4aS,1 1aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is present in the pellet at 99% by weight of drug loading.

The size and shape of the implant drug delivery systems may be modified to achieve a desired overall dosage.

The implant drug delivery systems described herein are capable of releasing (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide over a period of 21 days, 28 days, 31 days, 4 weeks, 6 weeks, 8 weeks, 12 weeks, one month, two months, three months, four months, five months, six months, seven months, eight months, nine months, ten months, eleven months, twelve months, eighteen months, twenty-four months or thirty-six months at an average rate of between 0.01-5 mg per day. In an embodiment of the invention, the (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is released at therapeutic concentrations for a duration from between three months and thirty-six months. In a class of the embodiment, the (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is released at therapeutic concentrations for a duration from between six months and twelve months.

The implant drug delivery systems described herein are capable of releasing (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide resulting in a plasma concentration of between 0.01-10 ng/mL per day. In an embodiment of the invention, the implant drug delivery systems described herein are capable of releasing (4aS,1 1aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide resulting in a plasma concentration of between 0.1-5.0 ng/mL per day. In a class of the embodiment, the implant drug delivery systems described herein are capable of releasing (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide resulting in a plasma concentration of between 0.1-2 ng/mL per day. In a subclass of the embodiment, the implant drug delivery systems described herein are capable of releasing (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide resulting in a plasma concentration of between 0.1-1.0 ng/mL per day.

The following examples are given for the purpose of illustrating the present invention and shall not be construed as being limitations on the scope of the invention.

### EXAMPLE 1

### Preparation of implant drug delivery systems containing (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide (active ingredient 1)

Implants were prepared by compression of a 99:1 w/w% active ingredient 1:magnesium stearate yielding pellets having diameters of 2.0 mm that were 7.5 mm +/- 0.2 mm in length. Dry, micronized powder of the active ingredient 1 was blended with magnesium stearate. The blend was granulated, and then compressed into pellets with 10 kN force and room temperature, with multi-tip round concave tablet tooling, to form tablet shaped pellets of 2 mm in diameter and approximately 50 mg each. Pellets were administered to rats and release rates were determined.

Three pellets were subcutaneously administered to each rat to achieve the desired amount of drug per implant for the target duration in the *in vivo* studies. For each implantation, a Wistar Han rat was anesthetized using isoflurane to effect prior to subcutaneous dose administrations. Using a trocar needle, the solid formulation (~2mm in diameter and a total of ~22.6 mm in length for each rat (3 pellets per rat) to achieve the dose appropriate for each group) was placed in the scapular region. Four animals (4 males) were used for each formulation. Animals were monitored until recovered. At indicated time points, samples of blood were obtained from anesthetized animals (using isoflurane) and processed to plasma for determination of active ingredient 1 levels.

The *in vitro* release rate of active ingredient 1 as 99% drug load compressed pellets was determined by placing the implants into a glass vial containing phosphate buffered saline (PBS+ 5 wt% solutol). The volume of PBS + 5 wt% solutol added was calculated such that if 100% of the drug was released, the concentration of active ingredient 1 would be equal to the maximum solubility, i.e., 0.26 mg/mL in PBS + 5 wt% solutol. The implant submerged in PBS + 5 wt% solutol was then added to an Innova 42 incubator shaker, maintained at 37°C, and 50 RPM shaking. Samples were removed at selected time points to assess the % active ingredient 1 released by HPLC over time (see Table 1). Release over time was measured in rats (see Table 2).

To compare the beneficial implant effects of pellets of the invention, an alternative formulation slurry was prepared having PEG 300 and active ingredient 1 in a PEG 300:active ingredient 1 ratio 1:2. The slurry was added to the interior of an extruded hydrophilic polyurethane tube (*in vitro* results in Table 3). The tubing had an outer diameter of 2.5 mm, and internal diameter of 2.1 mm. The hydrophilic polyurethane polymer had an equilibrium swelling of 20 wt% in water.

For the alternative PEG300 slurry inside the polyurethane tube formulation, the in vitro release rate of active ingredient 1 was determined by placing the implants into a glass vial containing phosphate buffered saline (PBS+ 2 wt% solutol). The volume of PBS + 2 wt% solutol added was calculated such that if 100% of the drug was released, the concentration of active ingredient 1 would be equal to 1/3 of the maximum solubility i.e. 0.15 mg/mL in PBS + 2 wt% solutol. The implant submerged in PBS + 2 wt% solutol was then added to an Innova 42 incubator shaker, maintained at 37°C, and 50 RPM shaking. Samples were removed at selected time points to assess the % active ingredient 1 released by HPLC over time (see Table 3). To generate *in vitro* release profiles of the various implants tested, 500 µL of dissolution media was removed from the sample vial at designated time points and centrifuged at 20,800xg for 8 min. The supernatant was removed (400 µL), diluted 4-fold, and vortexed. Samples were assayed by HPLC (Agilent 1100 series). Analysis of a 10 µL volume was performed at 210 nm with a YMCPack ODS-AQ column (50 x 4.6 mm, 5 µm). The mobile phase was 0.1% H₃PO₄:ACN (50:50 v/v) at a flow rate of 2.0 mL/min (40°C).

To determine degradation of active ingredient 1 by HPLC, a 5 µL volume was injected onto a Supelco Ascentis^{®} Express C18 column (100 x 4.6 mm, 2.7 µm). The mobile phase was 0.1% H₃PO₄ and ACN with a flow rate of 1.0 mL/min (40°C). The mobile phase gradient is shown below. All samples were calibrated to 0.2 mg/mL standard solutions of active ingredient 1 in 50:50 ACN: 0.1% H₃PO₄.

**Table 1**

| Active ingredient 1 *in vitro* release | | |
|---|---|---|
| 99 wt% active ingredient 1 compressed pellet | | |
| Time (days) | 99% active ingredient 1 compressed pellet | |
| | Average (% released) | Std. Dev. |
| 0 | 0 | 0 |
| 3 | 5 | 2 |
| 7 | 16 | 3 |
| 14 | 32 | 7 |
| 32 | 52 | 9 |

**Table 2**

| Active ingredient 1 *in vivo* release | | | | | | |
|---|---|---|---|---|---|---|
| 99 wt% active ingredient 1 compressed pellet in rats | | | | | | |
| Time (days) | 99% active ingredient 1 compressed pellet, plasma concentration (µM) | | | | | |
| | Animal #1 | Animal #2 | Animal #3 | Animal #4 | Average | Std. Dev. |
| 0.042 | 2.9 | 1.4 | 0.6 | 1.1 | 1 | 1 |
| 0.083 | 6.4 | 2.9 | 1.8 | 2.2 | 3 | 2 |
| 0.17 | 15.5 | 5.1 | 3.5 | 3.8 | 7 | 6 |
| 1 | 44.9 | 12.6 | 11.6 | 9.4 | 20 | 17 |
| 2 | 37.7 | 12.6 | 9.9 | 12.5 | 18 | 13 |
| 3 | 32.8 | 11.5 | 6.7 | 8.6 | 15 | 12 |
| 4 | 31.4 | 12.3 | 6.0 | 8.8 | 15 | 11 |
| 7 | 31.9 | 17.2 | 5.4 | 6.2 | 15 | 12 |
| 10 | 31.4 | 18.2 | 5.2 | 6.5 | 15 | 12 |
| 14 | 25.9 | 19.6 | 3.5 | 3.7 | 13 | 11 |
| 17 | 23.2 | 16.2 | 2.8 | 3.1 | 11 | 10 |
| 22 | 14.1 | 6.7 | 1.7 | 1.7 | 6 | 6 |
| 25 | 10.0 | 6.0 | 1.7 | 1.6 | 5 | 4 |
| 28 | 8.8 | 4.9 | 1.5 | 1.4 | 4 | 3 |
| 31 | 7.8 | 4.0 | 1.3 | 1.1 | 4 | 3 |
| 35 | 5.6 | 3.1 | 1.2 | 1.2 | 3 | 2 |
| 38 | 7.0 | 4.1 | 1.6 | 1.4 | 4 | 3 |
| 42 | 6.2 | 4.1 | 1.4 | 1.5 | 3 | 2 |
| 45 | 6.2 | 3.7 | 1.2 | 1.2 | 3 | 2 |
| 50 | 5.3 | 2.5 | 0.9 | 1.4 | 3 | 2 |
| 57 | 4.5 | 2.6 | 1.1 | 1.4 | 2 | 2 |
| 64 | 4.8 | 3.5 | 0.9 | 1.4 | 3 | 2 |
| 71 | 4.1 | 3.9 | 1.3 | 1.9 | 3 | 1 |
| 78 | 5.1 | 2.7 | 1.3 | 1.8 | 3 | 2 |
| 92 | 4.0 | 2.7 | 1.0 | 1.5 | 2 | 1 |
| 99 | 3.6 | 2.6 | 1.1 | 1.7 | 2 | 1 |
| 106 | 3.3 | 2.4 | 1.1 | 1.5 | 2 | 1 |
| 113 | 4.4 | 2.2 | 0.9 | 1.4 | 2 | 2 |
| 120 | 3.3 | 2.5 | 1.0 | 1.4 | 2 | 1 |
| 127 | 2.9 | 2.2 | 0.9 | 1.8 | 2.0 | 0.9 |
| 134 | 2.5 | 2.5 | 0.7 | 1.4 | 1.8 | 0.9 |
| 141 | 2.7 | 1.9 | 0.7 | 1.1 | 1.6 | 0.9 |
| 148 | 2.8 | 2.2 | 0.9 | 1.2 | 1.8 | 0.9 |
| 155 | 4.1 | 2.2 | 0.8 | 1.8 | 2 | 1 |
| 162 | 1.9 | 1.7 | 0.6 | 1.1 | 1.3 | 0.6 |
| 169 | 1.8 | 1.4 | 0.6 | 1.1 | 1.2 | 0.5 |
| 176 | 1.8 | 1.5 | 0.6 | 1.2 | 1.3 | 0.5 |
| 183 | 1.8 | 1.5 | 0.5 | 1.2 | 1.2 | 0.5 |
| 190 | 1.5 | 1.3 | 0.6 | 1.2 | 1.1 | 0.4 |

**Table 3**

| Active ingredient 1 *in vitro* release | | |
|---|---|---|
| 67 wt% active ingredient 1 slurry with 33% PEG 300 inside hydrophilic polyurethane tube | | |
| Time (days) | 67% active ingredient 1 | |
| | Average (% released) | Std. Dev. |
| 0 | 0 | 0 |
| 3 | 0.96 | 0.3 |
| 6 | 1.23 | 0.1 |
| 13 | 2.04 | 0.3 |
| 20 | 2.79 | 0.3 |
| 27 | 3.61 | 0.3 |
| 34 | 4.74 | 0.4 |
| 41 | 5.63 | 0.5 |
| 48 | 6.27 | 0.3 |
| 55 | 7.40 | 0.4 |
| 63 | 8.44 | 0.5 |
| 69 | 9.20 | 0.6 |
| 76 | 10.39 | 0.5 |

### EXAMPLE 2 (Reference)

### Preparation of implant drug delivery systems containing (3aS,5R)-N-(2,4-difluorobenzyl)-2-ethyl-8-hydroxy-5-methoxy-1,7-dioxo-2,3,3a,4,5,7-hexahydro-1H-pyrazino[2,1,6-cd]indolizine-6-carboxamide (active ingredient 2)

Implants were prepared by compression of a 99:1 w/w% active ingredient 2:magnesium stearate yielding pellets having diameters of 2.0 mm that were 7.5 mm +/- 0.2 mm in length. Dry, micronized powder of the active ingredient 1 was blended with magnesium stearate. The blend was granulated, and then compressed into pellets with 10 kN force and room temperature, with multi-tip round concave tablet tooling, to form tablet shaped pellets of 2 mm in diameter and approximately 50 mg each. Pellets were administered to rats and release rates were determined.

Three pellets were subcutaneously administered to each rat to achieve the desired amount of drug per implant for the target duration in the *in vivo* studies. For each implantation, a Wistar Han rat was anesthetized using isoflurane to effect prior to subcutaneous dose administrations. Using a trocar needle, the solid formulation (~2mm in diameter and a total of ~22.6 mm in length for each rat (3 pellets per rat) to achieve the dose appropriate for each group) was placed in the scapular region. Four animals (4 males) were used for each formulation. Animals were monitored until recovered. At indicated time points, samples of blood were obtained from anesthetized animals (using isoflurane) and processed to plasma for determination of active ingredient 2 levels.

The *in vitro* release rate of active ingredient 2 was determined by placing the implants into a glass vial containing phosphate buffered saline (PBS+ 2 wt% solutol). The volume of PBS + 2 wt% solutol added was calculated such that if 100% of the drug was released, the concentration of active ingredient 2 would be equal to the maximum solubility i.e. 0.31 mg/mL in PBS + 2 wt% solutol. The implant submerged in PBS + 2 wt% solutol was then added to an Innova 42 incubator shaker, maintained at 37°C, and 50 RPM shaking. Samples were removed at selected time points to assess the % active ingredient 2 released by HPLC over time (see Table 4). To generate *in vitro* release profiles of the various implants tested, 500 µL of dissolution media was removed from the sample vial at designated time points and centrifuged at 20,800xg for 8 min. The supernatant was removed (400 µL), diluted 4-fold, and vortexed. Samples were assayed by HPLC (Agilent 1100 series). Analysis of a 10 µL volume was performed at 210 nm with a YMCPack ODS-AQ column (50 x 4.6 mm, 5 µm). The mobile phase was 0.1% H₃PO₄:ACN (50:50 v/v) at a flow rate of 2.0 mL/min (40°C). Release over time was measured (see Table 5).

To determine degradation of active ingredient 2 by HPLC, a 5 µL volume was injected onto a Supelco Ascentis^{®} Express C18 column (100 x 4.6 mm, 2.7 µm). The mobile phase was 0.1% H₃PO₄ and ACN with a flow rate of 1.0 mL/min (40°C). The mobile phase gradient is shown in the table below. All samples were calibrated to 0.2 mg/mL standard solutions of active ingredient 2 in 50:50 ACN: 0.1% H₃PO₄.

**Table 4**

| Active ingredient 2 *in vitro* release | | |
|---|---|---|
| 99 wt% active ingredient 2 compressed pellets | | |
| Time (days) | 99% active ingredient 2 compressed pellet | |
| | Average (% release) | Std. Dev. |
| 0 | 0 | 0 |
| 3 | 10 | 1 |
| 7 | 21 | 1 |
| 14 | 33 | 1 |
| 32 | 42 | 1 |

**Table 5**

| Active ingredient 2 *in vivo* release | | | | | | |
|---|---|---|---|---|---|---|
| 99 wt% active ingredient 2 compressed pellets in rats | | | | | | |
| Time (days) | 99% active ingredient 2 compressed pellet, plasma concentration (µM) | | | | | |
| | Animal #1 | Animal #2 | Animal #3 | Animal #4 | Average | Std. Dev. |
| 0.042 | 0.201 | 0.566 | 0.542 | 0.681 | 0.50 | 0.21 |
| 0.083 | 0.294 | 0.801 | 0.738 | 1.12 | 0.74 | 0.34 |
| 0.17 | 0.317 | 0.786 | 0.56 | 1.22 | 0.72 | 0.38 |
| 1 | 0.065 | 0.151 | 0.142 | 0.371 | 0.18 | 0.13 |
| 2 | 0.0358 | 0.101 | 0.131 | 0.196 | 0.12 | 0.07 |
| 3 | 0.0447 | 0.0993 | 0.109 | 0.187 | 0.11 | 0.06 |
| 4 | 0.0351 | 0.068 | 0.123 | 0.186 | 0.10 | 0.07 |
| 7 | 0.023 | 0.0652 | 0.0815 | 0.127 | 0.074 | 0.043 |
| 10 | 0.0258 | 0.0613 | 0.0781 | 0.103 | 0.067 | 0.032 |
| 14 | 0.0289 | 0.057 | 0.074 | 0.0803 | 0.060 | 0.023 |
| 17 | 0.0223 | 0.0541 | 0.0543 | 0.0663 | 0.049 | 0.019 |
| 22 | 0.0213 | 0.0526 | 0.0594 | 0.0681 | 0.050 | 0.020 |
| 25 | 0.0242 | 0.0433 | 0.0482 | 0.0895 | 0.051 | 0.027 |
| 28 | 0.0194 | 0.0394 | 0.0493 | 0.0708 | 0.045 | 0.021 |
| 31 | 0.0195 | 0.0504 | 0.0557 | 0.0812 | 0.052 | 0.025 |
| 35 | 0.0196 | 0.0374 | 0.0447 | 0.0858 | 0.047 | 0.028 |
| 38 | 0.0255 | 0.0496 | 0.0477 | 0.114 | 0.059 | 0.038 |
| 42 | 0.0214 | 0.041 | 0.0469 | 0.0902 | 0.050 | 0.029 |
| 45 | 0.0183 | 0.0468 | 0.0303 | 0.0818 | 0.044 | 0.028 |
| 50 | 0.0183 | 0.0435 | 0.0333 | 0.0807 | 0.044 | 0.027 |
| 57 | 0.0178 | 0.0439 | 0.0296 | 0.0795 | 0.043 | 0.027 |
| 64 | 0.0157 | 0.0432 | 0.0303 | 0.0925 | 0.045 | 0.033 |
| 71 | 0.0166 | 0.041 | 0.0262 | 0.0722 | 0.039 | 0.024 |
| 78 | 0.0367 | 0.0143 | 0.0249 | 0.062 | 0.034 | 0.021 |
| 92 | 0.0129 | 0.0374 | 0.0233 | 0.0593 | 0.033 | 0.020 |
| 99 | 0.012 | 0.0372 | 0.024 | 0.0622 | 0.034 | 0.022 |
| 106 | 0.0123 | 0.0332 | 0.0214 | 0.0683 | 0.034 | 0.025 |
| 113 | 0.0132 | 0.0371 | 0.0163 | 0.0484 | 0.029 | 0.017 |
| 120 | 0.0105 | 0.0202 | 0.0155 | 0.0422 | 0.022 | 0.014 |
| 127 | 0.011 | 0.0264 | 0.021 | 0.0512 | 0.027 | 0.017 |
| 134 | 0.0145 | 0.0355 | 0.0191 | 0.0542 | 0.031 | 0.018 |
| 141 | 0.0158 | 0.0252 | 0.0146 | 0.0425 | 0.025 | 0.013 |
| 148 | 0.0096 | 0.03 | 0.0181 | 0.0601 | 0.029 | 0.022 |
| 155 | 0.0135 | 0.0309 | 0.0185 | 0.0486 | 0.028 | 0.016 |
| 162 | 0.0125 | 0.0312 | 0.0178 | 0.061 | 0.031 | 0.022 |
| 169 | 0.0138 | 0.0294 | 0.0185 | 0.0521 | 0.028 | 0.017 |
| 176 | 0.0148 | 0.0337 | 0.0208 | 0.0485 | 0.029 | 0.015 |
| 183 | 0.0131 | 0.0299 | 0.0165 | 0.0575 | 0.029 | 0.020 |
| 190 | 0.0113 | 0.0265 | 0.016 | 0.0569 | 0.028 | 0.020 |

## Claims

1. An implant drug delivery system comprising between 1 and 20 compressed pellets of (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide wherein the diameter of said pellet is about 1 mm to about 5 mm.

2. The implant drug delivery system of Claim 1 wherein the diameter of said pellet is about 2 mm to about 4 mm.

3. The implant drug delivery system of Claim 1 wherein the length of said pellet is about 5 mm to about 50 mm.

4. The implant drug delivery system of Claim 3 wherein the length of said pellet is about 7 mm to about 10 mm.

5. The implant drug delivery system of Claim 1 wherein the pellet is implanted subcutaneously and (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is continually released *in vivo* at a rate resulting in a plasma concentration between 0.5 ng/mL and 500 µg/mL.

6. The implant drug delivery system of Claim 5 wherein (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is continually released *in vivo* at a rate resulting in a plasma concentration between 50 ng/mL and 5000 ng/mL.

7. The implant drug delivery system of Claim 6 wherein (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is continually released *in vivo* at a rate resulting in a plasma concentration between 50 ng/mL and 11000ng/mL.

8. The implant drug delivery system of Claim 1 wherein (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is present at about 0.10% and 99% by weight of drug loading.

9. The implant drug delivery system of Claim 8 wherein (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is present at about 80% and 99% by weight of drug loading.

10. The implant drug delivery system of Claim 9 wherein (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is present at about 99% by weight of drug loading.

11. The implant drug delivery system of Claim 1 wherein the (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is released at therapeutic concentrations for a duration from between three months and thirty-six months.

12. The implant drug delivery system of Claim 11 wherein the (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide is released at therapeutic concentrations for a duration from between six months and twelve months.

13. The implant drug delivery system of Claim 1 comprising 1, 2 or 3 pellets.

14. An implant drug delivery system according to Claim 1 for use in treating HIV infection in a patient comprising subcutaneously implanting between 1 and 20 compressed pellets of (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide, wherein the diameter of said pellet is about 1 mm to about 5 mm.

15. An implant drug delivery system according to Claim 1 for use in preventing HIV infection in a patient comprising subcutaneously implanting between 1 and 20 compressed pellets of (4aS,11aR)-N-(2,4-difluorobenzyl)-6-hydroxy-4a-methyl-5,7-dioxo-2,3,4,4a,5,7,11,11a-octahydropyrano[3,2-b]quinolizine-8-carboxamide, wherein the diameter of said pellet is about 1 mm to about 5 mm.

## Patentansprüche

1. Ein Implantat-Arzneistoffverabreichungssystem, das zwischen 1 und 20 gepresste (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid-Pellets umfasst, wobei der Durchmesser des Pellets etwa 1 mm bis etwa 5 mm beträgt.

2. Das Implantat-Arzneistoffverabreichungssystem nach Anspruch 1, wobei der Durchmesser des Pellets etwa 2 mm bis etwa 4 mm beträgt.

3. Das Implantat-Arzneistoffverabreichungssystem nach Anspruch 1, wobei die Länge des Pellets etwa 5 mm bis etwa 50 mm beträgt.

4. Das Implantat-Arzneistoffverabreichungssystem nach Anspruch 3, wobei die Länge des Pellets etwa 7 mm bis etwa 10 mm beträgt.

5. Das Implantat-Arzneistoffverabreichungssystem nach Anspruch 1, wobei das Pellet subkutan implantiert wird und (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid kontinuierlich *in vivo* mit einer Rate, die zu einer Plasmakonzentration zwischen 0,5 ng/ml und 500 µg/ml führt, freigesetzt wird.

6. Das Implantat-Arzneistoffverabreichungssystem nach Anspruch 5, wobei (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid kontinuierlich *in vivo* mit einer Rate, die zu einer Plasmakonzentration zwischen 50 ng/ml und 5000 ng/ml führt, freigesetzt wird.

7. Das Implantat-Arzneistoffverabreichungssystem nach Anspruch 6, wobei (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid kontinuierlich *in vivo* mit einer Rate, die zu einer Plasmakonzentration zwischen 50 ng/ml und 11000 ng/ml führt, freigesetzt wird.

8. Das Implantat-Arzneistoffverabreichungssystem nach Anspruch 1, wobei (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid mit einer Wirkstoffbeladung von etwa 0,10 Gew.-% und 99 Gew.-% vorliegt.

9. Das Implantat-Arzneistoffverabreichungssystem nach Anspruch 8, wobei (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid mit einer Wirkstoffbeladung von etwa 80 Gew.-% und 99 Gew.-% vorliegt.

10. Das Implantat-Arzneistoffverabreichungssystem nach Anspruch 9, wobei (4aS,11aR)-N-(2,4-Difiuorbenzyl)-6-hydroxy-4a-methyl-5,7-dioxo-2,3,4,4a,5,7,11,11a-octahydropyrano[3,2-b]chinolizin-8-carboxamid mit einer Wirkstoffbeladung von etwa 99 Gew.-% vorliegt.

11. Das Implantat-Arzneistoffverabreichungssystem nach Anspruch 1, wobei das (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid in therapeutischen Konzentrationen für die Dauer von zwischen drei Monaten und sechsunddreißig Monaten freigesetzt wird.

12. Das Implantat-Arzneistoffverabreichungssystem nach Anspruch 11, wobei das (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid in therapeutischen Konzentrationen für die Dauer von zwischen sechs Monaten und zwölf Monaten freigesetzt wird.

13. Das Implantat-Arzneistoffverabreichungssystem nach Anspruch 1, umfassend 1, 2 oder 3 Pellets.

14. Ein Implantat-Arzneistoffverabreichungssystem nach Anspruch 1 zur Verwendung bei der Behandlung einer HIV-Infektion bei einem Patienten, umfassend das subkutane Implantieren von zwischen 1 und 20 gepressten (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid-Pellets, wobei der Durchmesser des Pellets etwa 1 mm bis etwa 5 mm beträgt.

15. Ein Implantat-Arzneistoffverabreichungssystem nach Anspruch 1 zur Verwendung bei der Prävention einer HIV-Infektion bei einem Patienten, umfassend das subkutane Implantieren von zwischen 1 und 20 gepressten (4aS,11aR)-N-(2,4-Difluorbenzyl)-6-hydroxy-4a-methyl-5,7-dioxo-2,3,4,4a,5,7,11,11a-octahydropyrano[3,2-b]chinolizin-8-carboxamid-Pellets, wobei der Durchmesser des Pellets etwa 1 mm bis etwa 5 mm beträgt.

## Revendications

1. Système d'administration de médicament pour implant comprenant entre 1 et 20 pastilles compressées de (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide, dans lequel le diamètre de ladite pastille est d'environ 1 mm à environ 5 mm.

2. Système d'administration de médicament pour implant selon la revendication 1, dans lequel le diamètre de ladite pastille est d'environ 2 mm à environ 4 mm.

3. Système d'administration de médicament pour implant selon la revendication 1, dans lequel la longueur de ladite pastille est d'environ 5 mm à environ 50 mm.

4. Système d'administration de médicament pour implant selon la revendication 3, dans lequel la longueur de ladite pastille est d'environ 7 mm à environ 10 mm.

5. Système d'administration de médicament pour implant selon la revendication 1, dans lequel la pastille est implantée par voie sous-cutanée et du (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est libéré de façon continue *in vivo* à une cadence engendrant une concentration plasmatique entre 0,5 ng/ml et 500 µg/ml.

6. Système d'administration de médicament pour implant selon la revendication 5, dans lequel du (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est libéré de façon continue *in vivo* à une cadence engendrant une concentration plasmatique entre 50 ng/ml et 5000 ng/ml.

7. Système d'administration de médicament pour implant selon la revendication 6, dans lequel du (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est libéré de façon continue *in vivo* à une cadence engendrant une concentration plasmatique entre 50 ng/ml et 11000 ng/ml.

8. Système d'administration de médicament pour implant selon la revendication 1, dans lequel le (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est présent dans une proportion d'environ 0,10 % à 99 % en poids de la charge de médicament.

9. Système d'administration de médicament pour implant selon la revendication 8, dans lequel le (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est présent dans une proportion d'environ 80 % à 99 % en poids de la charge de médicament.

10. Système d'administration de médicament pour implant selon la revendication 9, dans lequel le (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est présent dans une proportion d'environ 99 % en poids de la charge de médicament.

11. Système d'administration de médicament pour implant selon la revendication 1, dans lequel le (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est libéré à des concentrations thérapeutiques pendant une durée d'entre trois mois et trente-six mois.

12. Système d'administration de médicament pour implant selon la revendication 11, dans lequel le (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide est libéré à des concentrations thérapeutiques pendant une durée d'entre six mois et douze mois.

13. Système d'administration de médicament pour implant selon la revendication 1, comprenant 1, 2 ou 3 pastilles.

14. Système d'administration de médicament pour implant selon la revendication 1 pour utilisation dans le traitement de l'infection par le VIH chez un patient comprenant l'implantation par voie sous-cutanée d'entre 1 et 20 pastilles compressées de (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide, dans lequel le diamètre de ladite pastille est d'environ 1 mm à environ 5 mm.

15. Système d'administration de médicament pour implant selon la revendication 1 pour utilisation dans la prévention de l'infection par le VIH chez un patient comprenant l'implantation par voie sous-cutanée d'entre 1 et 20 pastilles compressées de (4aS,11aR)-N-(2,4-difluorobenzyl)-6-hydroxy-4a-méthyl-5,7-dioxo-2,3,4,4a,5,7,11,11a-octahydropyrano[3,2-b]quinolizine-8-carboxamide, dans lequel le diamètre de ladite pastille est d'environ 1 mm à environ 5 mm.
